# EUROPEAN PATENT APPLICATION

(11) **EP 3 885 455 A1**
(43) Date of publication of application: **29.09.2021**
(21) Application number: 20166524.7
(22) Date of filing: 27.03.2020
(51) Int. Cl.: C12Q 1/70

(54) **METHOD AND KIT FOR THE DETECTION OF SARS-COV-2 VIRUS IN A SAMPLE BASED ON REVERSE TRANSCRIPTION LOOP-MEDIATED ISOTHERMAL AMPLIFICATION (RT-LAMP)**

(71) Applicant: SKILLCELL, 97198 Jarry Cedex (FR); CENTRE NATIONAL DE LA RECHERCHE SCIENTIFIQUE -CNRS-, 75016 Paris (FR)
(72) Inventor: MOLINA, Franck, 34270 LES MATELLES (FR)
(74) Representative: Touroude, Magali Linda

(57) **Abstract**

The present invention is directed to a method for the detection of SARS-CoV-2 virus (COVID19 virus) based on reverse transcription loop-mediated isothermal amplification (RT-LAMP) designed to amplify specifically a sequence of SARS-CoV-2 gene fragment using a set of specially designed RT-LAMP primers. The present invention also relates to a kit for specifically detecting said sequence of of SARS-CoV-2 gene fragment comprising the set of specially designed RT-LAMP primers according to the present invention.

## Description

The present invention is directed to a method for the detection of SARS-CoV-2 virus (COVID19 virus) based on reverse transcription loop-mediated isothermal amplification (RT-LAMP) designed to amplify specifically a sequence of SARS-CoV-2 gene using a set of specially designed RT-LAMP primers. The present invention also relates to a kit for specifically detecting said sequence of of SARS-CoV-2 gene comprising the set of specially designed RT-LAMP primers according to the present invention.

The 2019 novel coronavirus COVID-19 now named SARS-CoV-2 virus is a newly emerged strain that has never been found in humans before. At present, the laboratory-based reverse transcription-polymerase chain reaction (RT-PCR) is the main method to confirm COVID-19 infection. In December 2019, the first case of cluster unidentified pneumonia was found in Wuhan, named 2019-Novel Coronavirus(2019-nCoV (or COVID-19) now named SARS-CoV-2 virus, a beta subtype, highly homologous with SARS. As of March 26, 2020, there were in total 480 000 confirmed cases worldwide. The intensification of the COVID-19 epidemic overwhelms limited clinical resources in particular, but not only, in developing countries, resulting in many patients not being tested for the infection and in large queues of potentially infected individuals waiting to be tested while providing a breeding ground for the disease. There is a heavy pressure for hospital assistant diagnosis, as some infected individuals without any symptoms could still transfer the virus to others ((Lin Yu et al. 2020 (https://doi.org/10.1101/2020.02.20.20025874).

The current diagnostic standard combines clinical symptoms and molecular method, and as many of the symptoms resembles those of common cold and influenza, an accurate molecular result is critical for final diagnosis. These molecular methods include metagenomics sequencing mNGS (1) and RT-qPCR(2), both are excellent and sensitive techniques, but approaches not without limitations. mNGS is restricted by throughput, turnover time, formidable high cost and requirement for high technical expertise. As with most molecular diagnostics, RT-qPCR is the most widely used method, but it requires expensive laboratory instruments and is difficult to utilize outside of well-equipped facilities. In combination to the different patient samples containing variable number of virus, a high proportion of patients were diagnosed as false negatives. For RNA virus infections, especially acute respiratory infection, probe coupled RT-qPCR from respiratory secretions is routinely used to detect causative viruses. This method has been widely used by Center for Disease Control and Prevention and other relevant departments worldwide. Recently RT-qPCR based methods were developed and applied worldwide by multiple research and disease control centers. However, RT-qPCR has many limitations such as the need for high purity samples and the access to expensive laboratory instruments, as well as requiring long reaction times (at least 2 h). In addition, RT-qPCR needs trained personnel and sophisticated facilities for sample processing. These disadvantages limit its practical application in many cases, and thus can delay the required rapid prescription and administration of antiviral agents to patients (Yinhua Zhang et al. 2020; https://doi.org/10.1101/2020.02.26.20028373)

Thus, there is an urgent need to develop new diagnostic method that is sensitive, accurate, rapid and low-cost to screen infected individuals, facilitate proper isolation and help controlling the disease spread.

Loop-mediated isothermal amplification (LAMP) is a technology that provides nucleic acid amplification in a short time using 4 to 6 specially designed primers and a DNA polymerase with chain displacement activity (Bst) under a constant temperature (60-65 °C). Particularly relevant for this application, is that LAMP can be combined with reverse transcription (i.e. RT-LAMP), were both reverse transcription and amplification occur simultaneously allowing the direct detection of RNA.

This system, can be coupled with a pH indicator present in the reaction mix allowing readout of the amplification reaction by change in color (Tanner NA et al., BioTechniques. 2015;58(2):59-68).

Therefore, RT- LAMP has the potential to offer an easy diagnostic test where the result could be directly observing the color change with the naked eye.

Surprisingly, it has been demonstrated that the two new target SARS-CO-V2 nucleic acid along with associated sets or combination thereof of RT-LAMP primers designed by the present inventors, allows to develop new diagnostic methods and kits that are sensitive, accurate, rapid and low-cost to screen infected individuals, particularly when said sets of primers are coupled with a naked-eye colorimetric pH dependent indicator present in the reaction mixture.

So, the present invention described a new RT-LAMP based method and kits for rapid detection of SARS-CoV-2, preferably by colorimetric detection.

This is the object of the present invention.

In a first aspect, the present invention is directed to a method of detecting the presence in a sample of SARS-CoV-2 nucleic acid based on a reverse transcription loop-mediated isothermal amplification (RT-LAMP), characterized in that it comprises the steps of:
a) contacting the sample with at least a combination of two sets of RT-LAMP primers specific for a SARS-CoV-2 target gene nucleic acid fragment under conditions sufficient for amplification of said SARS-CoV-2 target nucleic acid fragment, thereby producing SARS-CoV-2 target gene nucleic acid amplification product, wherein the at least combination of two sets of RT-LAMP primers comprises:
   - a first set of two primers named "F" set compring a forward outer primer (F3) and a a forward inner primer (FIP); and
   - a second set of two primers named "B" set compring a backward outer primer (B3) and a backward inner primer (BIP); and
b) detecting SARS-CoV-2 gene nucleic acid amplification product, thereby detecting presence of SARS-CoV-2 nucleic acid in the sample.
wherein the SARS-CoV-2 target gene nucleic acid fragment is selected from:
- The ORF3/Envelop protein (P) gene fragment having the sequence SEQ ID NO.1 (Genbank sequence MT111896.1), or sequence exhibiting 90% identity with the sequence SEQ ID NO.1, or specific fragment thereof;
- The Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
- The RdRp gene fragment having the sequence SEQ ID NO.3 (Genbank sequence MT159778.1)), or sequence exhibiting 90% identity with the sequence SEQ ID NO.3, or specific fragment thereof.

In another embodiment of the method of the present invention, in step a), the combination of two sets of RT-LAMP primers specific for a SARS-CoV-2 target gene nucleic acid fragment further comprised
- a third set of two primers comprisinf a forward loop primer (Loop F), and a backward loop primer (Loop B) associated to the two chosen sets "F" and "B".

Loop-mediated isothermal amplification (LAMP): A one temperature DNA amplification method that utilizes two or three pairs of primers, DNA strand displacement enzymes to catalyze the reaction. The method is a single-step amplification reaction utilizing a DNA polymerase with strand displacement activity (e.g., Notomi et al., Nucl. Acids. Res. 28:E63, 2000; Nagamine et al., Mol. Cell. Probes 16:223-229, 2002; Mori et al., J. Biochem. Biophys. Methods 59:145-157, 2004). The amplification reaction produces a stem-loop DNA with multiple inverted repeats of the target nucleic acid sequence. Reverse transcriptase can be added to the reaction for amplification of RNA target sequences, a process referred to as RT-LAMP.

Any nucleic acid or nucleotide sequence herein listed are shown using standard letter abbreviations for nucleotide bases and amino acids. In some cases, only one strand of each nucleic acid sequence is shown, while the complementary strand is understood as included by any reference to the displayed strand.

By primer, it is intended to designate short nucleic acids, generally DNA oligonucleotides 10 nucleotides or more in length (such as 10-60, 15-50, 20-45, or 20-40 nucleotides in length). Primers may be annealed to a complementary target DNA strand by nucleic acid hybridization to form a hybrid between the primer and the target DNA strand, and then extended along the target DNA strand by a DNA polymerase enzyme. Primer pairs can be used for amplification of a nucleic acid sequence, e.g., by the polymerase chain reaction (PCR), LAMP, RT-LAMP, or other nucleic acid amplification methods known in the art.

By probe, it is intended to designate a complementary nucleic sequence of about 10 or more nucleotides long and to which detectable molecules or label are attached such as for example fluorophore or fluorescent label.

By "Conditions sufficient for", it is intended to designate any environment that permits the desired activity, for example, that permits specific binding or hybridization between two nucleic acid molecules or that permits reverse transcription and/or amplification of a nucleic acid. Such an environment may include, but is not limited to, particular incubation conditions (such as time and or temperature) or presence and/or concentration of particular factors, for example in a solution (such as buffer(s), salt(s), metal ion(s), detergent(s), nucleotide(s), enzyme(s), and so on).

By "contact", it is intended to designate a placement in direct physical association; for example in solid and/or liquid form. For example, contacting can occur in vitro with one or more primers and/or probes and a biological sample (such as a sample including nucleic acids) in solution.

### SARS -CoV Severe Acute Respiratory Syndrome Coronavirus

SARS-CoV: Severe acute respiratory syndrome Coronavirus (SARS CoV) is an emerging infectious disease that has affected many countries since it was first discovered in Asia in 2003 (WHO. Wkly Epidemiol Rec;78:73-74, 2003). SARS CoV, which is transmitted through coughing out of respiratory droplets, is genetically related to the coronaviruses that are characterized by the presence of a single-stranded positive-sense RNA genome of about 30 kb in length (Marra et al., Science. 300:1399-1404, 2003; Drosten et al., N Engl J Med. 348:1967-1976, 2003). During the 2003 outbreak, SARS spread across five continents and had a cumulative total of 8464 cases and 799 deaths by May that year. SARS CoV sequences include GenBank Accession Nos.: FJ950407, AY545919, JX163925, and DQ497008 which are incorporated by reference as included in GenBank on Apr. 27, 2015. Additional SARS CoV species sequences can be identified by one skilled in the art.

Five SARS-CoV-2 sequences (MN908947, MN938384, MN988713, MN985325, and MN975262) and seven SARS-CoV sequences (NC_004718, AY613947, AY313906, AY559094, AY502924, AY278491, and AY502927) have been also cited by Gun-Soo Par et al. (https://doi.org/10.1101/2020.03.09.983064) and can be used whether it is necessary to confirm the specificity of the SARS-CoV-2 set of RT-LAMP primers of the present invention

### Middle East respiratory syndrome coronavirus (MERS CoV):

Middle East respiratory syndrome coronavirus (MERS-CoV) is a viral infection characterized by history of fever, cough, expectoration, and shortness of breath. It is a Coronavirus that was initially identified from a 60-year-old Saudi man who was admitted to a private hospital in Jeddah on Jun. 13, 2012 (Zaki et al., N Engl J Med. 367:1814-1820, 2012). Since the initial description, a total of 834 laboratory-confirmed cases of infection with MERS-CoV including 288 (34.5%) deaths were reported to the World Health Organization (WHO). MERS-CoV sequences include GenBank Accession Nos.: KF600647 and KF917527, both of which are incorporated by reference as included in GenBank on Apr. 12, 2015. Additional MERS-CoV species sequences can be identified by one skilled in the art.

In a preferred embodiment, said at least combination of two sets of RT-LAMP primers specific for a SARS-CoV-2 target gene nucleic acid fragment is selected from the group of combinations of two set of primers comprising:
A)
   - a first set of two primers named "F" set compring a forward outer primer (F3) and a a forward inner primer (FIP) selected from the group of combination primer SEQ ID IF to SEQ ID 9F, or a set of two primers having at least 90% identity with these primers ; and
   - a second set of two primers named "B" set compring a backward outer primer (B3) and a backward inner primer (BIP) selected from the group of combination primer SEQ ID 1B to SEQ ID 9B, or a set of two primers having at least 90% identity with these primers,
      when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
   - a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
   - a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
      when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
   - a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
   - a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
      when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

In a preferred embodiment, said SARS-CoV-2 gene nucleic acid amplification product is the fragment of the gene having the nucleic acid sequences SEQ ID NO. 1, 2 or 3, or a nucleic acid sequence thereof having at least 90% identity with the SEQ ID NO.1, 2 or 3 and specific for SARS-CoV-2 nucleic acid.

In a preferred embodiment, in the method of the present invention, the step a) comprises a step of placing the sample mixture on a thermal heating device for a predetermined period of time.

In a preferred embodiment, the predetermined period of amplification reaction time comprises a range of about 10 to 40 mn, preferably a range of about 15 to 25 mn .

In a preferred embodiment, in step a) of the claimed method, the sample mixture comprises a lysis buffer or a compound able to extract the total RNA from the cells contained in the sample, and incubated for about 2 to 10 minutes .

In a more preferred embodiment, said sample is a biological sample selected from the group consisting of blood, plasma, serum, urine, saliva, sperm, vaginal fluid, tissue biopsy, vomitus, fine needle aspirate, cerebrospinal fluid, bronchial aspirate, perspiration, mucus, fecal matter, synovial fluid, swabs, lymphatic fluid, tears, tracheal aspirate and/or a surgical specimen.

More preferably, the sample is a human sample.

In a preferred embodiment, the method of method of detecting the presence in a sample of SARS-CoV-2 nucleic acid further comprises a step of contacting the samples with a reverse transcriptase, DNA polymerase, deoxynucleotides, and, optionally RNAse inhibitor under conditions sufficient and optimal for synthesis and amplification of the SARS-CoV-2gene nucleic acid present in the sample.

In a more preferred embodiment, the method of detecting the presence in a sample of SARS-CoV-2 nucleic acid further comprises sample mixture comprises a pH indicator present in the reaction mix allowing readout of the amplification reaction by change in color, preferably a naked eye change of color.

In another embodiment, the method of detecting the presence in a sample of SARS-CoV-2 nucleic acid is characterized in that at least one primer in the set of RT-LAMP primers comprises a detectable label, preferably a fluorophore or fluorescent probe.

Detectable labels are detectable compounds such as fluorophores, radioactive isotopes, fluorescein, etc., that are conjugated to another molecule to enable detection, identification, and quantitation of said molecules. For example, nucleic acids are labeled for various purposes and several methods are available for facilitating the labeling of nucleic acids (Marras. Methods Mol Biol, 335: Mar. 16, 2006; Sambrook and Russell, in Molecular Cloning: A Laboratory Manual, 3.sup.rd Ed., Cold Spring Harbor Laboratory Press (2001).

In another aspect, the present invention relates to a method for the detection of SARS-CoV-2 based on a reverse transcription loop-mediated isothermal amplification (RT-LAMP), characterized in that it comprises the steps of:
a) providing a sample;
b) optionally extracting nucleic acid from the sample;
c) mixing the sample with amplification or detection reagents, wherein the reagents comprises at least a combination of two sets of RT-LAMP primers specific for a SARS-CoV-2 target gene nucleic acid;
d) optionally placing the sample mixture on a thermal heating device for a predetermined period of time;
e) amplifying the nucleic acid; and
f) detecting amplified products from said sample mixture obtained in step e), the presence of amplification product being significant of the presence of SARS-CoV-2 nucleic acid in the sample to be tested, and
wherein said at least combination of two sets of RT-LAMP primers specific for a SARS-CoV-2 target gene nucleic acid fragment is selected from the group of combinations of two set of primers comprising:
A)
   - a first set of two primers named "F" set compring a forward outer primer (F3) and a a forward inner primer (FIP) selected from the group of combination primer SEQ ID 1F to SEQ ID 9F, or a set of two primers having at least 90% identity with these primers ; and
   - a second set of two primers named "B" set compring a backward outer primer (B3) and a backward inner primer (BIP) selected from the group of combination primer SEQ ID 1B to SEQ ID 9B, or a set of two primers having at least 90% identity with these primers,
      when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
   - a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
   - a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
      when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
   - a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
   - a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
      when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

Methods for extracting nucleic acids such as RNA and/or DNA from a sample are known to one of skill in the art; such methods will depend upon, for example, the type of sample in which the nucleic acid is found. Nucleic acids can be extracted using standard methods. For instance, rapid nucleic acid preparation can be performed using a commercially available kit (such as kits and/or instruments from Qiagen (such as DNEasy.RTM. or RNEasy.RTM. kits), Roche Applied Science (such as MagNA Pure kits and instruments), Thermo Scientific (KingFisher mL), bioMerieux (Nuclisens.RTM. NASBA Diagnostics), or Epicentre (Masterpure.TM. kits)). In other examples, the nucleic acids may be extracted using guanidinium isothiocyanate, such as single-step isolation by acid guanidinium isothiocyanate-phenol-chloroform extraction (Chomczynski et al. Anal. Biochem. 162:156-159, 1987).

In a preferred embodiment, said SARS-CoV-2 amplification product is an amplification product of a gene nucleic acid, preferably the amplification product is the fragment of a gene having a nucleic acid sequences selected from the group of SEQ ID NO. 1, 2 and 3 or a nucleic acid sequence thereof having at least 90% identity with the SEQ ID NO.1, 2 and 3 and specific for SARS-CoV-2 nucleic acid.

In a second aspect, the present invention relates to a composition or a reagent comprising at least a combination of two sets of RT-LAMP primers specific for a SARS-CoV-2 target gene nucleic acid, wherein said at least combination of two sets of RT-LAMP primers specific for a SARS-CoV-2 target gene nucleic acid fragment is selected from the group of combinations of two set of primers comprising:
A)
   - a first set of two primers named "F" set compring a forward outer primer (F3) and a a forward inner primer (FIP) selected from the group of combination primer SEQ ID 1F to SEQ ID 9F, or a set of two primers having at least 90% identity with these primers ; and
   - a second set of two primers named "B" set compring a backward outer primer (B3) and a backward inner primer (BIP) selected from the group of combination primer SEQ ID 1B to SEQ ID 9B, or a set of two primers having at least 90% identity with these primers,
      when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
   - a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
   - a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
      when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
   - a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
   - a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
      when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

More preferred is a composition or reagent of the present invention, wherein the SARS-CoV-2 gene nucleic acid amplification product which can be obtained by said set of RT-LAMP primers is a the fragment of a gene having a nucleic acid sequences selected from the group of SEQ ID NO.1, 2 and 3 or a nucleic acid sequence thereof having at least 90% identity with the SEQ ID NO.1, 2 and 3 and specific for SARS-CoV-2 nucleic acid.

In a third aspect, the present invention relates to a kit for performing the method according to the present invention wherein the kit comprises the composition or reagents of the present invention as indicated above for the detection of SARS-CoV-2 virus in a sample, preferably in a biological sample from human origin.

The following examples, the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples and Figures, for which the legends are given hereinbelow.

### Figure legends:

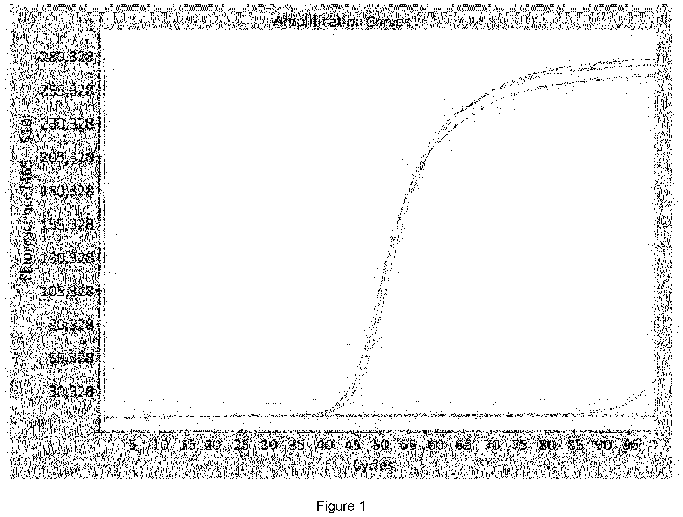

**Figure 1****:** Specific amplification of SARS-COV-2 cDNA using a combination of LAMP primers. (Left) Amplified sample was RNA from patients infected with SARS-COV-2 present in nasopharyngien mucus. Negative controls were performed with MERS, SARS-COV-1 and ultra pure water RNA free. (Right) Sharped-form curve demonstrates the specificity of the amplicon.

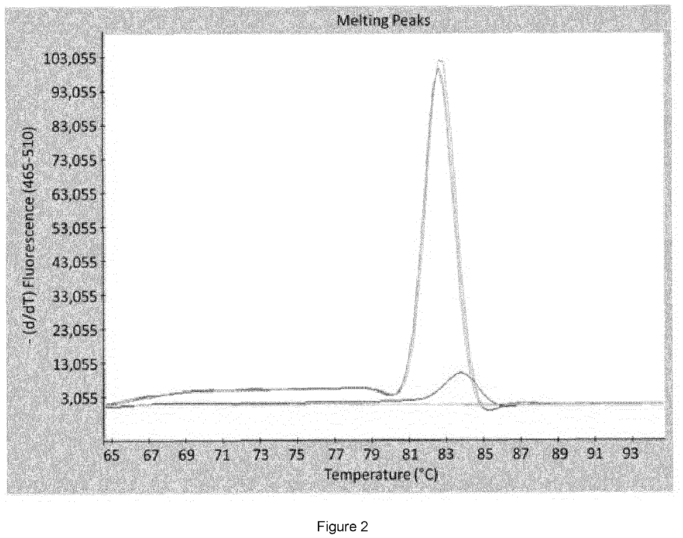

**Figure 2****:** Alignment of one targeted sequence with one example of primers' combination. Seq ID9.1 and Seq ID9.2 represent a F3 and FIP and Seq ID9.3 and Seq ID9.4 represent a B3 and BIP, respectively.

### EXAMPLE 1: MATERIALS AND METHODS

### Sample preparation

### Treatment of samples and System of detection

Saliva will be collected using an inoculation loop. This device allows the sampling of a small volume of saliva needed to RT-LAMP amplification (1µL approximately). The inoculation loop will be used to transfer saliva to a tube containing the Sputasol solution, for liquefying the saliva, and LAMP reaction mix.

The tube is then heated around 65°C for 45 minutes at max. This step allows the denaturation of viral capsid, liberation of viral RNA and the amplification of targeted sequences. This method allows an original LAMP single step reaction resulting in pH reduction which is detected by colorimetric change of the solution visible to the naked eye (red/pink to yellow in the case of a positive test).

### Materials and methods

### Target selection

Target gene sequences of SARS-CoV-2, and close related coronavirus species (SARS-CoV, MERS-CoV, ....), and other viral species such as H1N1 influenza virus ..., RSV, HPV... were downloaded from GenBank (https://www.ncbi.nlm.nih.gov/genbank/) to select the most specific target region.

Software was used for alignment analysis and to find the most specific region for designing the set of LAMP primers.

Software was used for primer design.

Designed primers were subjected to BLAST (https://blast.ncbi.nlm.nih.gov/Blast.cgi) and the specific candidates were used for analytical sensitivity and specificity testing

### Analytical sensitivity and specificity testing

In order to mimic the real viral particles, pseudotyped SARS-CoV-2 assay system containing target sequence were purchased.

RNA of pseudotyped virus were extracted using RNA Extraction Kit.

Serial dilutions with the magnitude of log10 containing 50*10⁶ cell/ml to 50*100 cell/ml pseudotyped virus were performed to determine the limit of detection (LOD). Serial dilution testing was performed in both RNase/DNase free molecular grade water and sputum sample collected from a SARS-CoV-2 negative healthy individual. Reproducibility of our LAMP assay (linearity=R2 value) was assessed by separate serial dilution testing on three occasions, each performed in duplicate. Signal intensity and the time to obtain decent amplification curves were recorded and R2 values ≥0.98 were considered reliable amplification.

Specificity testing included nucleic acid of (SARS-CoV, MERS-CoV, ....), and other viral species such as H1N1 influenza virus ..., RSV, HPV...

RT-LAMP incubation time was set to 60 minutes to observe both limit of detection and crossreactivity

(LAMP conditions are mentioned in clinical evaluation section). The reaction endpoint time was set in a way to detect the lowest possible copy number of virus without any cross-reaction.

### RT-LAMP Assays on Samples containing target RNA sequences from SARS-CoV-2 using the WarmStart® LAMP Kit (DNA & RNA) NEB #E1700S

The WarmStart® LAMP Kit (DNA & RNA) NEB #E1700S/L purchased from New England Biolabs, Inc. (Ipswich, Massachusetts, USA) is used.

For these experiments, the WarmStart LAMP Kit (DNA & RNA) is designed to provide a simple, one-step solution for Loop-Mediated Isothermal Amplification (LAMP) of RNA (RT-LAMP) targets(see Instruction Manual Version 2.0_1/20). RT-LAMP can commonly used isothermal amplification techniques that provides rapid detection of a target nucleic acid using LAMP-specific primers (supplied by the user) and a strand-displacing DNA polymerase.

This kit is supplied with the WarmStart LAMP 2X Master Mix, which contains a blend of Bst 2.0 WarmStart DNA Polymerase and WarmStart RTx Reverse Transcriptase in an optimized LAMP buffer solution. Both Bst 2.0 WarmStart DNA Polymerase and WarmStart RTx Reverse Transcriptase have been engineered for improved performance in RT-LAMP reactions.

A fluorescent dye is also supplied to enable real-time fluorescence measurement of LAMP whether this detection method is used.

The WarmStart LAMP Kit is also compatible with multiple detection methods, including turbidity detection, real-time fluorescence detection (when used with LAMP fluorescent dye) and end-point visualization.

For end-point visualization detection, this system can be for example coupled with a pH indicator present in the reaction mix allowing easily readout (naked-eye) of the amplification reaction by change in color (see Tanner NA et al., BioTechniques. 2015;58(2):59-68).

For example positive reactions resulted in a color change of phenol red pH indicator from pink to yellow due to decreased pH in the presence of extensive DNA polymerase activity. However, the negative reaction still kept pink.

### Warm Start RT-LAMP Kit Protocol used

The assay was performed in a 20 µl reaction mixture containing 2 µL of 10x primer mix of 16 µM (each) of Forward Inner Primer (FIP) and Backward Inner Primer (BIP), 2 µM (each) of F3 and B3 primers, 4 µM (each) of Forward Loop (LF) and Backward Loop (LB) primers, 10 µL of WarmStart Colorimetric Lamp 2X Master Mix (M1800)

5 µL of DNAse, RNAase free water (Beyotime Biotech, China),and 3 µl of RNA template. The reaction mixture was set at 65 °C for 30 minutes on a dry bath (Avist Technology Co., Wuhan, China).

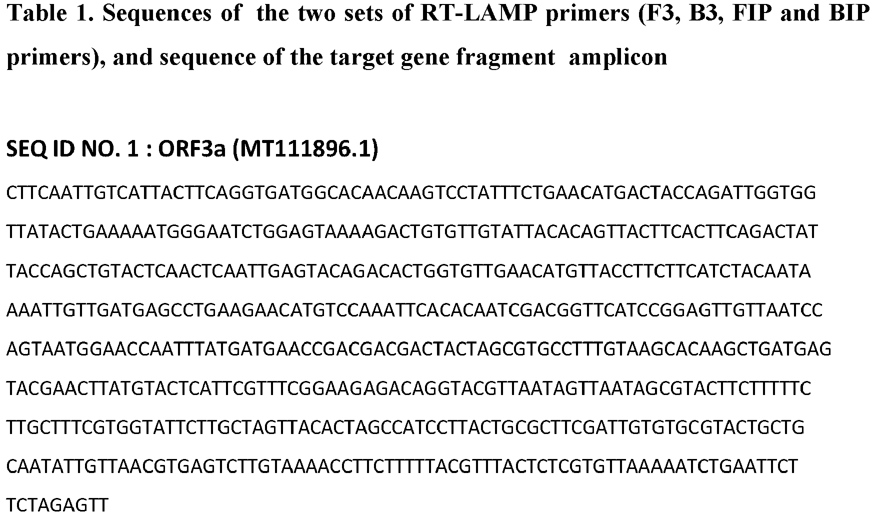

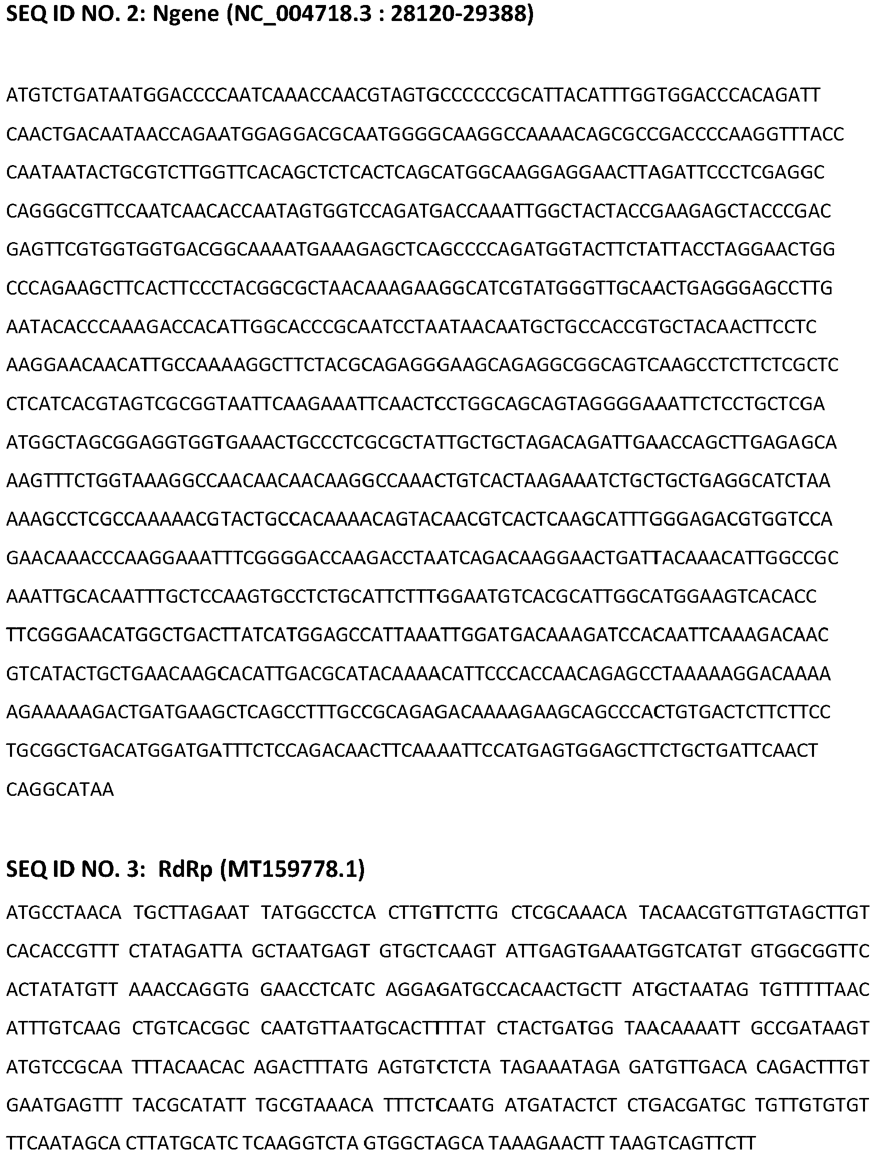

Set of RT-LAMP primers

| Seq ID number | type | **concerning SEQ ID NO.1: ORF3a (MT111896.1)** | Combination primer |
|---|---|---|---|
| | | Sequence | |
| Seq ID 1.1 (N0.4) | F3 | CACACAATCGACGGTTCA | Seq ID 1F |
| Seq ID 1.2(N0.5) | FIP | CGCTAGTAGTCGTCGTCGGTTTTTCCGGAGTTGTTAATCCAGT | |
| Seq ID 1.3(N0.6) | B3 | CAGTAAGGATGGCTAGTGT | Seq ID 1B |
| Seq ID 1.4(N0.7) | BIP | CTCATTCGTTTCGGAAGAGACAGGTTTTGCAAGAATACCACGAAAGC | |
| Seq ID 2.1(N0.8) | F3 | CACACAATCGACGGTTCA | Seq ID 2F |
| Seq ID 2.2(N0.9) | FIP | CGCTAGTAGTCGTCGTCGGTTTTTTCCGGAGTTGTTAATCCAG | |
| Seq ID 2.3(N0.10) | B3 | CAGTAAGGATGGCTAGTGT | Seq ID 2B |
| Seq ID 2.4(N0.11) | BIP | TCATTCGTTTCGGAAGAGACAGGTTTTGCAAGAATACCACGAAAGC | |
| Seq ID 3.1(N0.12) | F3 | ACCAGCTGTACTCAACTC | Seq ID 3F |
| Seq ID 3.2(N0.13) | FIP | GGACATGTTCTTCAGGCTCATCATTTTTTGAGTACAGACACTGGTG | |
| Seq ID 3.3(N0.14) | B3 | GCTTGTGCTTACAAAGGC | Seq ID 3B |
| Seq ID 3.4(N0.15) | BIP | TTCACACAATCGACGGTTCATCCTTTTTAGTAGTCGTCGTCGGTT | |
| Seq ID 4.1(N0.16) | F3 | AAAATTGTTGATGAGCCTGA | Seq ID 4F |
| Seq ID 4.2(N0.17) | FIP | CGTCGGTTCATCATAAATTGGTTCCTTTTAGAACATGTCCAAATTCACAC | |
| Seq ID 4.3(N0.18) | B3 | GCAAGAAAAAGAAGTACGCTAT | Seq ID 4B |
| Seq ID 4.4(N0.19) | BIP | GTGCCTTTGTAAGCACAAGCTGTTTTTAACGTACCTGTCTCTTCCG | |
| Seq ID 5.1(N0.20) | F3 | TGTAAGCACAAGCTGATGA | Seq ID 5F |
| Seq ID 5.2(N0.21) | FIP | CGAAAGCAAGAAAAAGAAGTACGCTTTTTTATGTACTCATTCGTTTCGGA | |
| Seq ID 5.3(N0.22) | B3 | AGAGTAAACGTAAAAAGAAGGTT | Seq ID 5B |
| Seq ID 5.4(N0.23) | BIP | TGGTATTCTTGCTAGTTACACTAGCTTTTAAGACTCACGTTAACAATATTGC | |
| Seq ID 6.1(N0.24) | F3 | CACTTCAGACTATTACCAGCT | Seq ID 6F |
| Seq ID 6.2(N0.25) | FIP | GTTCTTCAGGCTCATCAACAATTTTTTTTTACTCAACTCAATTGAGTACAGAC | |
| Seq ID 6.3(N0.26) | B3 | GCTTGTGCTTACAAAGGC | Seq ID 6B |
| Seq ID 6.4(N0.27) | BIP | CCAAATTCACACAATCGACGGTTTTTTTAGTAGTCGTCGTCGGTT | |
| Seq ID 7.1(N0.28) | F3 | TTCACACAATCGACGGTT | Seq ID 7F |
| Seq ID 7.2(N0.29) | FIP | CTAGTAGTCGTCGTCGGTTCATTTTCATCCGGAGTTGTTAATCCA | |
| Seq ID 7.3(N0.30) | B3 | GCAAGAAAAAGAAGTACGCTAT | Seq ID 7B |
| Seq ID 7.4(N0.31) | BIP | CGTGCCTTTGTAAGCACAAGCTTTTAACTATTAACGTACCTGTCTCTTC | |
| Seq ID 8.1(N0.32) | F3 | GTGTTGAACATGTTACCTTCT | Seq ID 8F |
| Seq ID 8.2(N0.33) | FIP | GGATGAACCGTCGATTGTGTGAATTTTTCTACAATAAAATTGTTGATGAGCC | |
| Seq ID 8.3(N0.34) | B3 | ACGAATGAGTACATAAGTTCGTA | Seq ID 8B |
| Seq ID 8.4(N0.35) | BIP | AGTTGTTAATCCAGTAATGGAACCATTTTCTCATCAGCTTGTGCTTAC | |
| Seq ID 9.1(N0.36) | F3 | CTTCAGGTGATGGCACAA | Seq ID 9F |
| Seq ID 9.2(N0.37) | FIP | CAGTCTTTTACTCCAGATTCCCATTTTTTCAAGTCCTATTTCTGAACATGAC | |
| Seq ID 9.3(N0.38) | B3 | TCTTCAGGCTCATCAACAA | Seq ID 9B |
| Seq ID 9.4(N0.39) | BIP | TTCACTTCAGACTATTACCAGCTGTTTTTGTAACATGTTCAACACCAGTG | |

| Seq_ID number | type | **Concerning SEQ ID NO. 2: Ngene (NC_004718.3 : 28120-29388)** | Combination primer |
|---|---|---|---|
| | | Sequence | |
| Seq ID 10.1 (N0. 41) | F3 | TGG CTA CTA CCG AAG AGC T | |
| Seq ID 10.2 (N0. 42) | FIP | TCT GGC CCA GTT CCT AGG TAG TTT TTG ACG AAT TCG TGG TGG TGA | Seq ID 10F |
| Seq ID 10.3 (N0. 43) | B3 | TGC AGC ATT GTT AGC AGG AT | |
| Seq ID 10.4 (N0. 44) | BIP | AGA CGG CAT CAT ATG GGT TGC ATT TTG CGG GTG CCA ATG TGA TC | Seq ID 10B |
| Seq ID 11.1 (N0. 45) | F3 | AGA TCA CAT TGG CAC CCG | |
| Seq ID 11.2 (N0. 46) | FIP | TGC TCC CTT CTG CGT AGA AGC TTT TCA ATG CTG CAA TCG TGC TAC | Seq ID 11F |
| Seq ID 11.3(N0. 47) | B3 | CCA TTG CCA GCC ATT CTA GC | |
| Seq ID 11.4( N0. 48) | BIP | GGC GGC AGT CAA GCC TCT TCT TTT CCT ACT GCT GCC TGG AGT T | Seq ID 11B |
| Seq ID 12.1 (N0. 49) | F3 | GCC AAA AGG CTT CTA CGC A | |
| Seq ID 12.2 (N0. 50) | FIP | TCC CCT ACT GCT GCC TGG AGT TTT GCA GTC AAG CCT CTT CTC G | Seq ID 12F |
| Seq ID 12.3 (N0. 51) | B3 | TTG CTC TCA AGC TGG TTC AA | |
| Seq ID 12.4 (N0. 52) | BIP | TCT CCT GCT AGA ATG GCT GGC ATT TTT CTG TCA AGC AGC AGC AAA G | Seq ID 12B |
| Seq ID 13.1 (N0. 53) | F3 | TGG ACC CCA AAA TCA GCG | |
| Seq ID 13.2 NO. 54) | FIP | CCA CTG CGT TCT CCA TTC TGG TTT TTA AAT GCA CCC CGC ATT ACG | Seq ID 13F |
| Seq ID 13.3(N0. 55) | B3 | GCC TTG TCC TCG AGG GAA T | |
| Seq ID 13.4 (N0. 56) | BIP | CGC GAT CAA AAC AAC GTC GGC TTT TCC TTG CCA TGT TGA GTG AGA | Seq ID 13B |
| Seq ID 14.1 (N0. 57) | F3 | CCA GAA TGG AGA ACG CAG TG | |
| Seq ID 14.2 (N0. 58) | FIP | AGC GGT GAA CCA AGA CGC AGT TTT GGC GCG ATC AAA ACA ACG | Seq ID 14F |
| Seq ID 14.3 (N0. 59) | B3 | CCG TCA CCA CCA CGA ATT | |
| Seq ID 14.4 (N0. 60) | BIP | AAT TCC CTC GAG GAC AAG GCG TTT TAG CTC TTC GGT AGT AGC CAA | Seq ID 14B |
| Seq ID 15. (N0. 61) | F3 | TGT CTG GTA AAG GCC AAC AA | |
| Seq ID 15.2 (N0. 62) | FIP | GTG GCA GTA CGT TTT TGC CGA GTT TTA ACA AGG CCA AAC TGT CAC T | Seq ID 15F |
| Seq ID 15.3(N0. 63) | B3 | GCA ATT TGC GGC CAA TGT | Seq ID 15B |
| Seq ID 15.4 (N0. 64) | BIP | ACA CAA GCT TTC GGC AGA CGTTTT TTG ATT AGT TCC TGG TCC CCA | |
| Seq ID 16.1 (N0. 65) | F3 | TGT CTG GTA AAG GCC AAC AA | Seq ID 16F |
| Seq ID 16.2 (N0. 66) | FIP | GTG GCA GTA CGT TTT TGC CGA GTT TTA ACA AGG CCA AAC TGT CAC T | |
| Seq ID 16.3 (N0. 67) | B3 | GCA ATT TGC GGC CAA TGT | Seq ID 16B |
| Seq ID 16.4 (N0. 68) | BIP | ACA CAA GCT TTC GGC AGA CGT TTT TTG ATT AGT TCC TGG TCC CCA | |

| Seq_ID number | type | **Concerning SEQ ID NO. 3: RdRp (MT159778.1)** | Combination primer |
|---|---|---|---|
| | | Sequence | |
| Seq ID 17.1(N0. 69) | F3 | AAT GTA GTG CGT ATA AAG ATT GT | Seq ID 17F |
| Seq ID 17.2(N0. 70) | FIP | AGC CAT GTG CCC ATA AGA CATTTT TAA GTG ACA CAC TTA AAA ATC TCT C | |
| Seq ID 17.3(N0. 71) | B3 | ACA GGC ATA AGT GTC TGA AG | Seq ID 17B |
| Seq ID 17.4 (N0. 72) | BIP | ATT TTG TGA AAA TAG GAC CTG AGC GTT TTC AGT GGA AAA GCA TGT GG | |
| Seq ID 18.1 (N0. 73) | F3 | CCT TGG AAT GTA GTG CGT A | Seq ID 18F |
| Seq ID 18.2 (N0. 74) | FIP | GCC CAT AAG ACA AAT ACG ACT CTG TTT TTA GAT TGT ACA AAT GTT AAG TGA CAC | |
| Seq ID 18.3 (N0. 75) | B3 | AAG CAG TGG AAA AGC ATG | Seq ID 18B |
| Seq ID 18.4 (N0. 76) | BIP | ACA TGG CTT TGA GTT GAC ATC TTT TTC ACG TCT ATC ACA TAG ACA ACA | |
| Seq ID 19.1 (N0. 77) | F3 | TGT ACA AAT GTT AAG TGA CAC A | Seq ID 19F |
| Seq ID 19.2 (N0. 78) | FIP | AGA TGT CAA CTC AAA GCC ATG TGT TTT CTT AAA AAT CTC TCT GAC AGA GTC | |
| Seq ID 19.3 (N0. 79) | B3 | GCC AAC AGG CAT AAG TGT | Seq ID 19B |
| Seq ID 19.4 (N0. 80) | BIP | ATT TTG TGA AAA TAG GAC CTG AGC GTT TTC AGT GGA AAA GCA TGT GG | |
| Seq ID 20.1 (N0. 81) | F3 | ACC ACT TAT GTA CAA AGG ACT | Seq ID 20F |
| Seq ID 20.2 (N0. 82) | FIP | GAC AAA TAC GAC TCT GTC AGA GAG ATT TTT CCT TGG AAT GTA GTG CG | |
| Seq ID 20.3 (N0. 83) | B3 | GTG GAA AAG CAT GTG GCA | Seq ID 20B |
| Seq ID 20.4 (N0. 84) | BIP | GCA CAT GGC TTT GAG TTG ACA TTT TTC TAT CAC ATA GAC AAC AGG TG | |
| Seq ID 21.1 (N0. 85) | F3 | ACA AAT GTT AAG TGA CAC ACT | Seq ID 21F |
| Seq ID 21.2 (N0. 86) | FIP | CGC TCA GGT CCT ATT TTC ACA AAA TTT TTC TGA CAG AGT CGT ATT TGT CTT | |
| Seq ID 21.3 (N0. 87) | B3 | AAT CAT AAA CGG ATT ATA GAC GT | Seq ID 21B |
| Seq ID 21.4 (N0. 88) | BIP | CAC CTG TTG TCT ATG TGA TAG ACG TTT TAA TCC AAT AGA ATG ATG CCA AC | |

### RESULTS AND DISCUSSION

### RT-LAMP Reaction Optimization

To optimize RT-LAMP reaction we first evaluated optimal concentration/condition /temperature/ lysis buffer: detergent.

### Assessing Limit of Detection and Cross-Reactivity

The limit of detection of the RT-LAMP method targeting the new target gene fragment according to the present invention and according to the method of the present invention could as low RNA concentration per reaction.

The cross-reactivity of SARS-CoV-2 RT-LAMP method targeting the new target gene fragment according to the present invention and according to the method of the present invention was not found for SARS-CoV and MERS-CoV

### Detection of amplified product by alternative methods

To further expand the detection capability of the method of the present invention, the UV or Blue light stimulated fluorescence signal can be also checked. SYBR green dye can be added into the reaction mix prior to adding the sample. After i.e. about 20 minutes incubation in 65 °C , the signal can be observed with Gel imaging system (. SYBR green in positive reactions can be observed clearly in positive reaction. We also chose A new type of nucleic acid dye GeneFinderTM, which has enhanced fluorescent signal and sensitivity can be also chosen. By exposing under Blue light, green fluorescence can be observed clearly with naked eye in the positive reaction, whereas it remained original pink in the negative control.

### Discussions and Conclusion

In this study, we designed LAMP primer sets targeting SARS-CoV-2.

### LOD

Reaction optimization was also performed for selected primer sets. In summary, we designed and evaluated RT-LAMP assays for detection of SARS-CoV-2 with limit of detection of copies per reaction.

### Specificity

Our RT-LAMP assays showed specificity to SARS-CoV-2 versus other human Betacoronavirus because proper sample was not in our hand, specificity of the RT-LAMP assays are easily expectable from the mismatching bases in primer binding sites, especially, both of F1/B1 sites of new target gene fragment primer set is in SARS-CoV-2 specific region of which aligning SARS-CoV sequence is not exist. The blast analysis suggest that the set of Lamp primers which have designed have a low chance for unspecific amplifications (false positives).

### Incubation time/temperature

The results of the RT-LAMP method of the present invention can be detected within 30 minutes around 65°C after amplification reaction begin at 65°C.

### Detection system

In addition, the RT-LAMP method of the present invention is optimized reaction conditions to which easy colorimetric detection method is applied that can be used for point-of-care tests.

### Single-step assy

In addition, not only purified RNA can be used as the sample input, but also direct tissue or cell lysate may be used without an RNA purification step.

This combination of a quick sample preparation method with an easy one-step detection process may allow the development of portable, field detection in addition to a rapid screening for point-of-need testing applications, and usable for screening tests.

In conclusion, by designing new sets and combination of set of RT-LAMP primers targeting the region of new target gene fragments, the inventors have developed a new diagnostic method and kit for detection of SARS-CoV-2 that is sensitive, specific, accurate, rapid and low-cost to screen infected individuals, facilitate proper isolation and help controlling.

## Claims

1. A method of detecting the presence in a sample of SARS-CoV-2 nucleic acid based on a reverse transcription loop-mediated isothermal amplification (RT-LAMP), **characterized in that** it comprises the steps of:
a) contacting the sample with at least a combination of two sets of RT-LAMP primers specific for a SARS-CoV-2 target gene nucleic acid fragment under conditions sufficient for amplification of said SARS-CoV-2 target nucleic acid fragment, thereby producing SARS-CoV-2 target gene nucleic acid amplification product, wherein the at least combination of two sets of RT-LAMP primers comprises:
- a first set of two primers named "F" set compring a forward outer primer (F3) and a a forward inner primer (FIP); and
- a second set of two primers named "B" set compring a backward outer primer (B3) and a backward inner primer (BIP); and
b) detecting SARS-CoV-2 gene nucleic acid amplification product, thereby detecting presence of SARS-CoV-2 nucleic acid in the sample.
wherein the SARS-CoV-2 target gene nucleic acid fragment is selected from:
- The ORF3/Envelop protein (P) gene fragment having the sequence SEQ ID NO.1 (Genbank sequence MT111896.1), or sequence exhibiting 90% identity with the sequence SEQ ID NO.1, or specific fragment thereof;
- The Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
- The RdRp gene fragment having the sequence SEQ ID NO.3 (Genbank sequence MT159778.1)), or sequence exhibiting 90% identity with the sequence SEQ ID NO.3, or specific fragment thereof.

2. The method of claim 1, wherein said at least combination of two sets of RT-LAMP primers specific for a SARS-CoV-2 target gene nucleic acid fragment is selected from the group of combinations of two set of primers comprising:
A)
- a first set of two primers named "F" set compring a forward outer primer (F3) and a a forward inner primer (FIP) selected from the group of combination primer SEQ ID 1F to SEQ ID 9F, or a set of two primers having at least 90% identity with these primers ; and
- a second set of two primers named "B" set compring a backward outer primer (B3) and a backward inner primer (BIP) selected from the group of combination primer SEQ ID 1B to SEQ ID 9B, or a set of two primers having at least 90% identity with these primers,
when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
- a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
- a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
- a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
- a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

3. The method of one of claims 1 and 2, wherein said SARS-CoV-2 gene nucleic acid amplification product is a fragment of a gene, said gene having a nucleic acid sequences selected from the group of SEQ ID NOs. 1, 2 and 3, or a nucleic acid sequence thereof having at least 90% identity with the SEQ ID NOs.1, 2 and 3 and specific for SARS-CoV-2 nucleic acid.

4. The method of one of claims 1 of 3, wherein the step a) comprises a step of placing the sample mixture on a thermal heating device for a predetermined period of time.

5. The method of one of claims 1 of 4, wherein the predetermined period of amplification reaction time comprises a range of about 10 to 40 mn, preferably a range of about 15 to 25 mn .

6. The method of one of claims 1 of 5, wherein in step a) the sample mixture comprises a lysis buffer or a compound able to extract the total RNA from the cells contained in the sample, and incubated for about 2 to 10 minutes.

7. The method of one of claims 1 of 6, wherein the sample comprises blood, plasma, serum, urine, saliva, sperm, vaginal fluid, tissue biopsy, vomitus, fine needle aspirate, cerebrospinal fluid, bronchial aspirate, perspiration, mucus, fecal matter, synovial fluid, swabs, lymphatic fluid, tears, tracheal aspirate and/or a surgical specimen.

8. The method of one of claims 1 of 7, further comprising: contacting the samples with a reverse transcriptase, DNA polymerase, deoxynucleotide, and, optionally RNAse inhibitor under conditions sufficient and optimal for synthesis and amplification of the SARS-CoV-2 gene nucleic acid present in the sample.

9. The method of one of claims 1 of 8, wherein the sample mixture comprises a pH indicator present in the reaction mix allowing readout of the amplification reaction by change in color, preferably a naked eye change of color.

10. The method of one of claims 1 of 9, wherein at least one primer in the set of LAMP primers comprises a detectable label, preferably a fluorophore or fluorescent probe.

11. A method for the detection of SARS-CoV-2 based on a reverse transcription loop-mediated isothermal amplification (RT-LAMP), **characterized in that** it comprises the steps of:
a) providing a sample;
b) optionally extracting nucleic acid from the sample;
c) mixing the sample with amplification or detection reagents, wherein the reagents comprises at least a combination of two sets of RT-LAMP primers specific for a SARS-CoV-2 target gene nucleic acid;
d) optionally placing the sample mixture on a thermal heating device for a predetermined period of time;
e) amplifying the nucleic acid; and
f) detecting amplified products from said sample mixture obtained in step e), the presence of amplification product being significant of the presence of SARS-CoV-2 nucleic acid in the sample to be tested, and
wherein said at least combination of two sets of RT-LAMP primers specific for a SARS-CoV-2 target gene nucleic acid fragment is selected from the group of combinations of two set of primers comprising:
A)
- a first set of two primers named "F" set compring a forward outer primer (F3) and a a forward inner primer (FIP) selected from the group of combination primer SEQ ID 1F to SEQ ID 9F, or a set of two primers having at least 90% identity with these primers ; and
- a second set of two primers named "B" set compring a backward outer primer (B3) and a backward inner primer (BIP) selected from the group of combination primer SEQ ID 1B to SEQ ID 9B, or a set of two primers having at least 90% identity with these primers,
when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
- a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
- a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
- a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
- a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

12. The method of claim 11, wherein said SARS-CoV-2 gene nucleic acid amplification product is a fragment of a gene, said gene having a nucleic acid sequences selected from the group of SEQ ID NOs. 1, 2 and 3, or a nucleic acid sequence thereof having at least 90% identity with the SEQ ID NOs.1, 2 and 3 and specific for SARS-CoV-2 nucleic acid.

13. A composition or a reagent comprising at least least a combination of two sets of RT-LAMP primers specific for a SARS-CoV-2 target gene nucleic acid, wherein said at least combination of two sets of RT-LAMP primers specific for a SARS-CoV-2 target gene nucleic acid fragment is selected from the group of combinations of two set of primers comprising:
A)
- a first set of two primers named "F" set compring a forward outer primer (F3) and a a forward inner primer (FIP) selected from the group of combination primer SEQ ID 1F to SEQ ID 9F, or a set of two primers having at least 90% identity with these primers ; and
- a second set of two primers named "B" set compring a backward outer primer (B3) and a backward inner primer (BIP) selected from the group of combination primer SEQ ID 1B to SEQ ID 9B, or a set of two primers having at least 90% identity with these primers,
when the SARS-CoV-2 target gene nucleic acid fragment is selected from the ORF3/Envelop protein (P) gene fragment having the sequence SEQ ID NO.1 or sequence exhibiting 90% identity with the sequence SEQ ID NO.1, or specific fragment thereof;
B)
- a first set of two primers named "F" set compring a forward outer primer (F3) and a a forward inner primer (FIP) selected from the group of combination primer SEQ ID 10F to SEQ ID 16F, or a set of two primers having at least 90% identity with these primers ; and
- a second set of two primers named "B" set compring a backward outer primer (B3) and a backward inner primer (BIP) selected from the group of combination primer SEQ ID 10 B to SEQ ID 16B, or a set of two primers having at least 90% identity with these primers,
when the SARS-CoV-2 target gene nucleic acid fragment is selected from the Ngene gene fragment having the sequence SEQ ID NO.2 (Genbank sequence NC_004718.3 : 28120-29388), or sequence exhibiting 90% identity with the sequence SEQ ID NO.2, or specific fragment thereof; and
C)
- a first set of two primers named "F" set compring a forward outer primer (F3) and a a forward inner primer (FIP) selected from the group of combination primer SEQ ID 17F to SEQ ID 21F, or a set of two primers having at least 90% identity with these primers ; and
- a second set of two primers named "B" set compring a backward outer primer (B3) and a backward inner primer (BIP) selected from the group of combination primer SEQ ID 17 B to SEQ ID 21B, or a set of two primers having at least 90% identity with these primers,
when the SARS-CoV-2 target gene nucleic acid fragment is selected from the RdRp gene fragment having the sequence SEQ ID NO.3 (Genbank sequence MT159778.1)), or sequence exhibiting 90% identity with the sequence SEQ ID NO.3, or specific fragment thereof.

14. The composition or reagent of claims 13, wherein the SARS-CoV-2 gene nucleic acid amplification product which can be obtained by said set of RT-LAMP primers is a the fragment of a gene having a nucleic acid sequences selected from the group of SEQ ID NO. 1, 2 and 3 or a nucleic acid sequence thereof having at least 90% identity with the SEQ ID NO.1, 2 and 3 and specific for SARS-CoV-2 nucleic acid.

15. A kit for performing the method according to one of claims 1 to 12, wherein the kit comprises the composition or reagents of one of claims 13 or 14.
